Europäisches Patentamt

(19) European Patent Office

Office européen des brevets

(11) Publication number: **0 442 962 B1**

# EUROPEAN PATENT SPECIFICATION

(45) Date of publication of patent specification: **05.01.94** (51) Int. Cl.5: **A61K 9/127, A61K 49/00**

(21) Application number: **90900449.1**

(22) Date of filing: **08.11.89**

(86) International application number:
**PCT/US89/05040**

(87) International publication number:
**WO 90/04943 (17.05.90 90/11)**

The file contains technical information submitted
after the application was filed and not included in
this specification

(54) **LIPOSOMAL RADIOLOGIC CONTRAST AGENTS.**

(30) Priority: **09.11.88 US 269190**
　　　　　**27.10.89 US 428339**

(43) Date of publication of application:
**28.08.91 Bulletin 91/35**

(45) Publication of the grant of the patent:
**05.01.94 Bulletin 94/01**

(84) Designated Contracting States:
**AT BE CH DE FR GB IT LI LU NL SE**

(56) References cited:
**EP-A- 0 160 552　　EP-A- 0 272 091**
**WO-A-86/00238　　US-A- 4 532 089**
**US-A- 4 652 257　　US-A- 4 675 173**
**US-A-41 928 59**

(73) Proprietor: **UNGER, Evan C**
**13365 E. Camino La Cebadilla**
**Tucson, AZ 85749(US)**

Proprietor: **TILCOCK, Colin**
**3592 Dunbar Street**
**Vancouver, British Columbia V6S 2C5(CA)**

(72) Inventor: **UNGER, Evan C**
**13365 E. Camino La Cebadilla**
**Tucson, AZ 85749(US)**
Inventor: **TILCOCK, Colin**
**3592 Dunbar Street**
**Vancouver, British Columbia V6S 2C5(CA)**

(74) Representative: **Stebbing, Peter John Hunter**
**et al**
**Ablett & Stebbing**
**45 Lancaster Mews**
**Lancaster Gate**
**London W2 3OO (GB)**

EP 0 442 962 B1

A JOURNAL OF CLINICAL AND LABORATO-
RY RESEARCH, INVESTIGATIVE RADIOLOGY,
vol. 23, no. 10, October 1988, pages 719-724,
J.B. Lippincott Co., Philadelphia, PA, US; J.M.
DEVOISSELLE et al.: "Entrapment of
gadolinium-DTPA in liposomes characteriza-
tion of vesicles by P-31 NMR spectroscopy"

A JOURNAL OF CLINICAL AND LABORATO-
RY RESEARCH, INVESTIGATIVE RADIOLOGY,
vol. 22, no. 1, January 1987, pages 47-55, J.B.
Lippincott Co., Philadelphia, PA, US; K.T.
CHENG et al.: "The production and evalu-
ation of contrast-carrying liposomes made
with an automatic high-pressure system"

JOURNAL OF CLINICAL AND LABORATORY
RESEARCH, INVESTIGATIVE RADIOLOGY,
vol. 23, suppl. 1, September 1988, pages
S122-S125, J.B. Lippincott Co., Philadelphia,
PA, US; S.E. SELTZER: "Contrast-carrying
liposomes current status"

RADIOLOGY, vol. 171, no. 1, April 1989,
pages 77-80, Easton, PA, US; C. TILCOCK et
al.: "Liposomal Gd-DTPA: Preparation and
characterization of relaxivity"

JOURNAL OF LIPOSOME RESEARCH, vol. 1,
no. 1, 1988/89, pages 137-150, New York, US;
C.P.S. TILCOCK et al.: "Magnetic filtration of
vesicles containing iron-dextran particles"

Biochimica et. Biophysica Acta, vol. 858,
1986, Mayer et al "Vesicles of variable sizes
produced by a rapid extrusion procedure",
pp. 161-168

Biochimica et Biophysica Acta, vol. 812
(1985) Hope et al. "Production of large un-
ilamellar vesicles by a rapid extruision pro-
cedure", pp. 55-65

J. Pharm. Pharmacol., vol. 39 (1987) Higgins
et al "A comparative investigation of
glycinebetaine and DMSO as liposome
cryoprotectives", pp. 577-582

2

## Description

Accurate detection of the presence, location and extent of splenic and hepatic tumors is of profound clinical significance. With recent advancements in oncological therapy, many malignant tumors can be effectively treated if their existence is promptly and correctly ascertained. Over the years, the medical profession has relied on various imaging methods in an effort to provide such diagnoses. Many of these imaging methods, however, have disadvantages and/or limitations associated with their use.

Radionuclide scans, for example, were used early on for the detection of malignancy within the liver and spleen. Such scans rely on the uptake of particulates, such as technetium sulphur colloid, by the Kupffer cells of the liver and spleen. Resolution, however, is poor, and the use of this procedure for malignancy detection has been limited.

Computed tomography (CT), an imaging method that provides high spatial resolution, has been used extensively for the detection of liver and splenic malignancy. CT relies on the differential linear attenuation of tumors and adjacent normal hepatic or splenic tissue (parenchyma). Generally malignancies have a somewhat lower attenuation than adjacent parenchyma, and are thus detectable on CT. In some cases, however, tumors show similar attenuation as the parenchyma and, as such, are not visible upon non-contrast CT examination.

To improve the detection of liver and splenic tumors on CT, iodinated water soluble contrast agents have been employed. These iodinated compounds are distributed throughout the extracellular fluid and lead to the detection of approximately 16% additional malignancies. Such contrast agents, however, rely upon blood flow for delivery. Therefore, a hepatic or splenic tumor with similar blood flow as surrounding tissue often is not detected using contrast enhanced CT. Overall, only about 70% of hepatic metastases are detected using this method, and for malignant lymphomas, the detection rate is even poorer. Lymphomatous involvement of the spleen is also extremely difficult to detect on CT, with or without contrast media, this method having at best a sensitivity of only about 50% where splenic lymphoma is involved.

The disappointing level of improvement observed with the extracellular iodinated contrast media led to the search for tissue specific contrast agents for use in CT detection of hepatic and splenic tumors. Much of the effort focused on either oily contrast media such as ethiodol (EOE-13) or particulate contrast agents such as microencapsulated or liposomally encapsulated iodinated contrast media. These oily or particulate iodinated contrast agents are accumulated preferentially by normal cells of the liver, spleen and bone marrow, particularly by Kupffer cells. Clinical trials have shown that these agents improve CT detectability, especially where lymphoma identification is concerned. CT, however, requires a relatively high dose of contrast agent (in the amount of about 10 millimolar iodine) to provide differential contrast and, unfortunately, such contrast agents have proven toxic at this dosage. Thus, while helpful in improving contrast, problems such as toxicity have restricted the use of CT employing these tissue specific contrast agents to certain centers and experimental trials.

Magnetic resonance imaging (MRI) has proven to be the most useful detection technique for identifying hepatic and splenic malignancies. In MRI, tumors are detected because of a difference between the relaxation times ($T_1$ and/or $T_2$) of normal and malignant tissue. This difference in $T_1$ and/or $T_2$ causes differential signal intensity (contrast) on MR images, with greater differences in $T_1$ (or $T_2$), leading to greater contrast. Typically, liver tumors have a longer $T_1$ and a longer $T_2$ than corresponding normal tissue. This results in tumors showing a hypointensity on $T_1$-weighted MR images and a hyperintensity on $T_2$-weighted MR images, relative to surrounding parenchyma. In some instances, however, malignancies and normal tissue may be isointense on MRI, and therefore nondetectable.

In an attempt to lower the instances of isointensity and increase the overall detection rate of this imaging method, agents capable of enhancing tissue and tumor contrast were sought. The major MRI agent developed as a result of this effort was the paramagnetic compound gadolinium diethylenetriamine pentaacetic acid (Gd-DTPA), discussed in Weinmann et al., A.J.R., Vol. 142, pp. 619-624 (1985); Brasch et al., A.J.R., Vol. 142, pp. 625-630 (1984); and Strich et al., Radiology, Vol. 154, pp. 723-726 (1985). Under certain conditions, free Gd-DTPA is capable of providing good contrast enhancement in the micromolar range, approximately two orders of magnitude lower in concentration than required for iodinated contrast enhancement on CT. When effectively employed, Gd-DTPA results in concentration dependent decreases in either normal tissue $T_1$ and $T_2$ or tumor $T_1$ and $T_2$, but not both. The higher the concentration of contrast agent that reaches the tissue, the greater the measured effect on $T_1$ and $T_2$. The measured $T_1$ (or $T_2$) is given by the formula

$1/T_1$ (measured) = $1/T_1$ (tissue) + $1/T_1$ (contrast)

where $1/T_1$ (tissue) is the $T_1$ in tissue in the absence of contrast agent and $1/T_1$ (contrast) is the contribution to tissue $T_1$ caused by the contrast agent. As the foregoing formula shows, the relaxation rates are additive. Gadian et al., A.J.R., Vol. 143, pp. 215-224 (1985); and Brown et al., Med. Phys., Vol. 11, pp. 67-72 (1984).

Unfortunately, human trials with free Gd-DTPA evaluating its use in diagnoses involving the liver and spleen have been disappointing. One problem is that free Gd-DTPA, like the iodinated contrast media employed in CT, is an extracellular agent which is distributed throughout the extracellular fluid space proportional to blood flow. Because the relaxation rates are additive, the effect of contrast agent is proportionately greater on tissues with longer relaxation times than for tissues with shorter relaxation times. Tumors tend to exhibit longer relaxation times than normal tissue. Damadian et al., Science, Vol. 171, pp. 1151-1153 (1971). Accordingly, if the contrast agent equilibrates between tumor and normal tissue, the effect will be to decrease the tumor $T_1$ (and $T_2$) more than the normal tissue $T_1$ (and $T_2$), which will result in a decrease in the contrast between the tissues. Thus, where tumors have similar blood flow as surrounding tissue, the tumor $T_1$ (and $T_2$) is also affected, and such tumors often go undetected.

Research into increasing the effectiveness of Gd-DTPA as an MRI contrast agent has focused on the use of liposomes to encapsulate and selectively deliver Gd-DTPA to liver and splenic parenchyma. Liposomes are cleared principally by the liver, spleen and bone marrow, and thus should be accumulated preferentially by such tissues. The primary collecting cells within these tissues are the phagocytic Kupffer cells. Tumors, however, generally do not contain such cells. Thus, theoretically, liposomes can be employed to selectively target contrast agents to normal hepatic, splenic and bone marrow tissue, without regard to blood flow, thereby increasing the contrast between the liver, spleen or bone marrow and any tumor cells.

A number of methods are available in the art to produce liposomes. For example, unilamellar lipid vesicles can be prepared from organic solvents by reverse phase techniques which involve either solvent dilution (Deamer et al., Biochim. Biophys. Acta, Vol. 443, pp. 629-634 (1976); and Kremer et al., Biochemistry, Vol. 16, pp. 3932-3935 (1977)) or solvent evaporation (Szoka et al., Proc. Natl. Acad. Sci., Vol. 79, pp. 4194-4198 (1978)). These procedures suffer from the common limitation that not all lipids are equally soluble in all organic solvents, thus the solvents employed must be varied when the lipid composition is altered (Schieren et al., Biochim. Biophys. Acta, Vol 542, pp. 137-153, 1978); and Deamer in Liposome Technology, Vol. 1 (CRC Press 1984) (Greguriadis, G., ed.)). Additionally, there is a need to remove residual solvents from the lipid preparations by chromatographic or dialysis techniques. The vesicles produced by such methods tend to be a heterogenous mixture of multilamellar and unilamellar vesicles. Thus, it is necessary to size the vesicles using various techniques such as by extrusion through polycarbonate filters. Olson et al., Biochim. Biophys. Acta, Vol. 557, pp. 9-23 (1978). U.S. Patent No. 4,752,425 discloses liposomes produced by such solvent dilution and extrusion techniques. In addition, U.S. Patent No. 4,737,323 describes a size-processing method for heterogeneous liposome suspensions prepared from various techniques. The processing described involves passing a suspension of the heterogeneously sized liposomes, wherein the liposome sizes are predominantly greater than about one micron, through an asymmetric ceramic filter having an inner surface pore size of about one micron.

Detergent removal represents another general method of liposome vesicle formation. In such methods, either the lipid materials or the pre-formed vesicles are solubilized in detergent to form mixed micelles. The detergent is then removed by dialysis and the micelles coalesce to form bilayer vesicles. One problem with this technique is that the particular dialysis method which can be effectively employed is heavily dependent upon the specific lipids being used. For example, different lipids may require the use of different lipid to detergent ratios (Mimms et al., Biochemistry, Vol. 20, pp. 833-840 (1981); Enoch et al., Proc. Natl. Acad. Sci., Vol. 76, pp. 145-149 (1979); and Weder et al., in Liposome Technology, Vol. 1, (CRC Press 1984) (Gregoriadis, G., ed.)) and the rate of detergent removal by dialysis or by gel filtration may need to be changed in order to obtain reproducible preparations (Mimms et al., Biochemistry, Vol. 20, pp. 833-840 (1981); and Schuh et al., Biochim. Biophys. Acta, Vol. 687, pp. 219-225 (1982)). Another major problem with this technique is the difficulty in removing all the residual detergent (Madden, Chem. Phys. Lipids, Vol. 40, pp. 207-221 (1986)).

Alternatively, unilamellar lipid vesicles may be prepared from multilamellar vesicles (or frozen and thawed multilamellar vesicles) by mechanical techniques involving cavitation and shearing (french press) (Lelkes, in Liposome Technology, Vol. 1, pp. 51-65 (CRC Press 1984) (Greguriadis, G., ed.)), sonication (Mayhew et al., in Liposome Technology, Vol. 2, pp. 19-31 (CRC Press 1984) (Greguriadis, G.)), or filter extrusion (Mayer et al., Biochim. Biophys. Acta., Vol. 858, pp. 161-168 (1986)) methods.

As those familiar with the art would recognize, the aforementioned sonication method has several limitations. First, it is difficult to make sonication a completely reproducible procedure. This is due in part to the fact that sonication involves the transfer of energy from the transducer to the medium to be sonicated.

The efficiency of energy transfer between the transducer and material to be sonicated depends upon many factors. These include the concentration of the lipid dispersion to be sonicated, the ionic strength of the medium in which the lipid is dispersed, whether a probe or bath-type sonicator is used, as well as other factors. In the case of sonication involving physical contact between the probe tip of the sonicator device and the material to be sonicated, the physical positioning of the probe tip within the dispersion can influence the sonication process. In cases where the medium to be sonicated is physically separate from the energy transducer of the sonicator, e.g., in a bath-type sonicator, the energy transfer will be modulated by the geometry and wall thickness of the vessel containing the material to be sonicated. It is also well recognized that it is difficult to employ the sonication process at lipid concentrations above about 100 mg/ml and achieve uniform size distributions. Since lipid concentrations are low, the trapping efficiency for any water-soluble solute will also tend to be low. Another disadvantage is that it is necessary to sonicate for long times in order to achieve uniform size distributions. Furthermore, in the process of sonication, the energy applied to the sonicate is liberated partially as heat. It is therefore necessary to cool the sample under sonication to avoid thermal degradation of the labile lipids. Moreover, it is also a difficult procedure to scale up. When using probe-type sonicators, for example, there is the possibility of contamination of sonicate with metallic particles from the tip of the probe. This must either be tested for or else the sonicate centrifuged to remove such particles from the sonicate, thus complicating the preparatory procedure further. Lastly, it has been reported that lipid vesicles prepared by sonication are unstable and tend to fuse (Parente et al., Biochemistry, Vol. 23, pp. 2353-2362 (1984)). This is undesirable since it is known that the overall size of lipid vesicles influences their biodistribution (Cullis et al., in Liposomes, pp. 39-72 (Marcel Dekker 1983) (Ostro, M., ed.)).

The french press method noted above involves the extrusion of lipid through a small orifice under high (approx. 20,000 psi) pressure. The procedure is rapid and versatile, but is, however, limited to relatively low lipid concentrations (< 50 mg/ml) which limits the trapping efficiency of the technique.

Related to the aforementioned french press method is the process of extruding lipid dispersions through filters of defined pore size. Mayer et al., Biochim. Biophys. Acta, Vol. 858, pp. 161-168 (1986). International Application PCT/US85/01161 describes such extrusion techniques as suitable for preparing liposomes of various lipid combinations having a substantially unimodal size distribution and a unilamellar construction. The unimodal size distribution results from repeatedly passing previously prepared liposomes through one or more filters each having the same pore size. The unilamellar construction is achieved by employing a filter having a pore size of about 100 nm or less. The preparation of liposomes directly from lipid pellets or powder and buffer, thus avoiding the addition of any extraneous materials such as solvents, detergents, etc., and the use of freeze-thaw cycles prior to extrusion to increase the trapped volumes of the liposomes, are also discussed. The liposomes thus prepared are described as useful in delivering entrapped material in vivo. Mayer et al., Biochimica et Biophysica Acta, Vol. 858, pp. 161-168 (1986), Hope et al., Biochimica et Biophysica Acta, Vol. 812, pp. 55-65 (1985) and Mayer et al., Biochimica et Biophysica Acta, Vol. 817, pp. 193-196 (1985) contain disclosures similar to PCT/US/01161.

A few of the foregoing methods have been employed to produce Gd-DTPA-containing liposomes for use as contrast agents, and such efforts have been reported in the literature. For example, Unger et al., Radiology, Vol. 157(P), p. 314 (1985) (abstract) (presented at the 71st Radiologic Society of North America Scientific Assembly, Chicago, Illinois, November 19, 1985), examines the use of Gd-DTPA containing liposomes prepared by sonication methods to selectively deliver contrast media to the liver and spleen.

U.S. Patent No. 4,728,575 discloses liposomal vesicles containing paramagnetic materials such as Gd-DTPA which are useful as contrast agents for NMR imaging. According to the patent, the Gd-DTPA-containing vesicles can be prepared using methods such as homogenization, chelate dialysis, sonication, and the like. External Gd-DTPA can be removed from the vesicles by gel filtration, ultrafiltration or similar methods. In the preparatory example shown, the paramagnetic vesicles were formed using sonication methods followed by gel filtration. The disclosures suggest that the liposomal contrast agent described is targeted to tumor, rather than the normal, tissue, which, if true, would theoretically cause a decrease, not an increase, in contrast between normal and tumor tissue, particularly when employed in the liver.

U.K. Patent Application GB 2193095 A, published February 3, 1988, discusses liposomes containing macromolecule-bound paramagnetic ions such as Gd-DTPA and their use as NMR contrast agents. The liposomes described in the examples were prepared using a sonication-type process.

Despite the theoretical reasons why the liposomally encapsulated contrast agents prepared to date and reported in such publications as Unger et al., Radiology, Vol. 157(P), p. 314 (1985) (abstract) (presented at the 71st Radiologic Society of North America Scientific Assembly, Chicago, Illinois, November 19, 1985), U.S. Patent No. 4,728,575 and U.K. Patent Application GB 2193095 A, should solve the need for acceptable MRI contrast agents for use in detecting hepatic and splenic malignancies, there are in fact substantial problems and/or limitations associated with their use. First, contrast enhancement (differential relaxivity) observed with these products is lower than desirable. In addition, such liposomes have been found to be relatively unstable and prone to leakage of the paramagnetic contrast agents. Moreover, the encapsulation efficiency of the aforementioned liposome/contrast agent systems developed to date is quite low. Furthermore, and quite significantly, toxicity problems have been encountered. These, as well as other disadvantages, have made the use of such liposomally encapsulated paramagnetic agents less desirable than had been hoped.

It has now been found in the present invention that liposomes prepared by a freeze-thaw extrusion process and having encapsulated therein a paramagnetic and/or superparamagnetic agent such as Gd-DTPA provide a significantly better and more useful MRI contrast agent than heretofore known in the art. These contrast agents provide high relaxivity and good contrast enhancement, resulting in a concomitant increase in tumor detection rate. Moreover, these agents show no observable toxicity and are extremely stable, retaining their integrity even after long periods of storage.

It has also been found in the present invention that liposomes prepared as above and having a diameter of less than about 50 nm and having encapsulated therein a paramagnetic and/or superparamagnetic agent provide excellent MRI contrast agents. Surprisingly, these small sized liposomes exhibit superior relativity and contrast enhancement, resulting in a significant increase in tumor detection, and are also particularly effective as blood pool imaging agents.

## SUMMARY OF THE INVENTION

The present invention provides for the use of contrast agents for magnetic resonance imaging (MRI) which are particularly useful as blood pool imaging agents and in diagnosing the presence of tumorous tissue in the liver and spleen.

Specifically, the present invention provides for the use in the manufacture of an MRI contrast agent of liposomes having encapsulated therein a paramagnetic and/or superparamagnetic agent, prepared by a freeze-thaw extrusion process or a microemulsification process to have a diameter of less than 50nm, as an MRI contrast agent.

The present invention is useful in a method for diagnosing the presence of tumorous tissue in a patient comprising (i) administering to the patient one or more of the foregoing MRI contrast agents, and (ii) scanning the patient using MRI to obtain visible images of any tumorous tissue. Further the present invention is also useful as a method of imaging the blood pool of a patient comprising (i) administering to the patient liposomes and (ii) scanning the patient using MRI to obtain visual images of the location of the blood pool.

The subject invention is described in greater detail below.

## DETAILED DESCRIPTION OF THE INVENTION

The present invention provides for the use of liposomes having encapsulated therein a paramagnetic and/or superparamagnetic agent, prepared by a freeze-thaw extrusion process or a microemulsification process to have a diameter of less than 50 nm, as an MRI contrast agent.

The term "freeze-thaw extrusion" as used herein, denotes the use of a process wherein liposomes are subjected to one or more cycles of freezing and thawing, followed by one or more passages through an extruder. Specifically, the freeze-thaw extrusion process may be carried out as follows.

First, liposomes containing paramagnetic and/or super-paramagnetic agents are initially prepared using any one of a variety of conventional liposome preparatory techniques. As will be readily apparent to those skilled in the art, such conventional techniques include sonication, chelate dialysis, homogenization, solvent infusion coupled with extrusion, as well as others. These techniques, as well as others, are discussed, for example, in U.S. Patent No. 4,728,578, U.K. Patent Application GB 2193095 A, U.S. Patent No. 4,728,575, U.S. Patent No. 4,737,323, International Application PCT/US85/01161, Mayer et al., Biochimica et Biophysica Acta, Vol. 858, pp. 161-168 (1986), Hope et al., Biochimica et Biophysica Acta, Vol. 812, pp. 55-65 (1985), U.S. Patent No. 4,533,254, Mayhew et al., Methods in Enzymology, Vol. 149, pp. 64-77 (1987), Mayhew et al., Biochimica et Biophysica Acta, Vol. 775, pp. 169-174 (1984) and Cheng et al., Investigative Radiology, Vol. 22, pp. 47-55 (1987). As a preferred technique, a solvent free system similar to that described in International Application PCT/US85/01161, is employed in initially preparing the liposome constructions. Using this technique, a lipid powder or pellet is placed in an aqueous buffer solution containing the paramagnetic and/or superparamagnetic agents of the present invention.

6

The liposomes thus prepared are then subjected to at least one freeze-thaw cycle, that is, freezing followed by thawing. In the freezing portion of the cycle, the liposomes are placed in suitable vials which are then subjected to liquid nitrogen, preferably for about 30 seconds to about 5 minutes. Although liquid nitrogen is the preferred cooling medium, other cooling procedures may also be used. For example, the vials with liposomes may be placed in a freezer at a temperature of preferably about -20°C to about -90°C for preferably up to about one hour. Moreover, cooling mixtures such as acetone/dry ice, methanol/dry ice or ethanol/dry ice may be used used as an alternative to liquid nitrogen.

The thawing portion of the cycle is carried out by transferring frozen liposome-containing vials from the cooling medium, to a medium having a temperature greater than about 0°C. Typically, the vials are placed in warm water between about 30°C and about 70°C. The temperature is not critical to the thawing, but it is important that the vials are held at the elevated temperature until the liposome suspension has completely thawed.

This entire cycle of freezing and thawing is performed at least one and up to about ten times, or more.

After completion of the freeze-thaw cycle(s), the thawed liposomes are then passed at least one and up to about twenty times, or more, under pressure through an extruder having a filter with pore size of about 15 to about 600 nm diameter. More than one filter may be used, in, for example, a series alignment. The pressure may be applied in the form of nitrogen, argon, or other inert gas, or, alternatively, may be applied by mechanical pump or direct displacement. As one skilled in the art would recognize, the size of the filter pore determines the relative size, lamellarity, trap volume and size distribution of the resultant lipid vesicles. As will be apparent to those in the art, the smaller the pore size, the smaller the liposome size, trap volume and size distribution, and the less the lamellarity. It should be noted that the application of heat to the extruder device, either from an external source (heat plate, heat gun or the like) or by means of a thermostatted jacket, renders the lipid membranes more deformable and allows extrusion to take place at lower pressures. In addition, by applying such heat, smaller sized vesicles can be more easily produced. It is preferable to extrude lipid vesicles at temperatures at which their lipids are in the liquid-crystal rather than gel state, to facilitate extrusion of the liposomes. Suitable temperatures will be readily apparent to those skilled in the art, by reference to standard lipid texts.

Although filter pore sizes of 15 to 600 nm may be employed, preferable filter pore sizes are 15 to 30 nm which will generally yield liposomes in the range of 20 to 50 nm in diameter. More preferably, pore sizes of 15 to 25 nm, generally yielding liposomes of 20 to 30 nm in diameter, most preferably pore sizes of about 25 nm, generally yielding liposomes of about 35 nm in diameter, are utilized. The smaller pore sizes are preferred since they yield correspondingly smaller liposomes as well as liposomes with correspondingly less lamellarity. It has been found that the smaller liposomes exhibit greater relaxivity and/or show greater effectiveness at improving contrast. This greater relaxivity is believed to be due to the presence of a proportionally larger surface area relative to the internal volume, which permits a greater diffusion of water across the lipid membrane and thus better contrast enhancement. Significantly, relaxivity has been found to bear a direct linear relationship with 1/r, wherein r is radius. In addition, the smaller liposomes have been found to exhibit longer circulation half-lives. Further, it has been found that the less lamellar liposome vesicles, particularly the unilamellar liposome vesicles, also have been found to exhibit a higher relaxivity, presumably because, with less lamellarity, a higher rate of water transport across the lipid membrane can also occur.

If desired, the liposomes can be extruded, then freeze-thawed, in addition to being extruded subsequent to the freeze-thaw cycle. The entire freeze-thaw extrusion process can be carried out as many times as desirable. Freeze-thaw extrusion techniques as generally employed in the preparation of liposomes are known in the art, and are described, for example, in International Application PCT/US85/01161, Mayer et al., Biochimica et Biophysica Acta, Vol. 858, pp. 161-168 (1986), and Hope et al., Biochimica et Biophysica Acta, Vol. 812, pp. 55-65 (1985). Such techniques may be utilized in the preparation of the freeze-thawed paramagnetic and superparamagnetic encapsulating liposomes of the present invention.

Encapsulation techniques are discussed, for example, in U.S. Patent No. 4,728,578, U.K. Patent Application GB 2193095 A, U.S. Patent No. 4,728,575, U.S. Patent No. 4,737,323, International Application PCT/US85/01161, Mayer et al., Biochimica et Biophysica Acta, Vol. 858, pp. 161-168 (1986), Hope et al., Biochimica et Biophysica Acta, Vol. 812, pp. 55-65 (1985), U.S. Patent No. 4,533,254, Mayhew et al, Methods in Enzymology, Vol. 149, pp. 64-77 (1987), Mayhew et al., Biochimica et Biophysica Acta, Vol. 775, pp. 169-74 (1984), and Cheng et al, Investigative Radiology, Vol. 22, pp. 47-55 (1987). The techniques of the invention for the preparation of liposomes having a diameter of less than 50 nm require a solvent free freeze-thaw extrusion technique or a microemulsification technique because of their respective suitability for large scale production of liposomal radiologic contrast agents.

Freeze-thaw procedures have been described in detail herein, and include those procedures described, for example, in International Application PCT-US85/01161, Mayer et al., Biochimica et Biophysica Acta, Vol. 858, pp. 161-168 (1986), and Hope et al., Biochimica et Biophysica Acta, Vol. 812, pp. 55-65 (1985)

Microemulsification techniques are discussed in U.S. Patent No. 4,533,254, Mayhew et al., Methods in Enzymology, Vol. 149, pp. 64-77 (1987), Mayhew et al., Biochimica et Biophysica Acta, Vol. 775, and Cheng et al., Investigative Radiology, Vol. 22, pp. 47-55 (1987).

Specifically, the microemulsion process may be carried out as follows.

First, liposomes containing paramagnetic and/or superparamagnetic agents are initially prepared using any one of a variety of conventional liposomes preparatory techniques. As will be readily apparent to those skilled in the art, such conventional techniques include sonication, chelate dialysis, homogenization, solvent infusion coupled with extrusion, as well as others. These techniques, as well as others, are discussed, for example, in U.S. Patent No. 4,728,578, U.K. Patent Application GB 2193095 A, U.S. Patent No. 4,728,575, U.S. Patent No. 4,737,323, International Application PCT/US85/01161, Mayer et al., Biochimica et Biophysica Acta, Vol. 858, pp. 161-168 (1986), Hope et al., Biochimica et Biophysica Acta, Vol. 812, pp. 55-65 (1985), U.S. Patent No. 4,533,254, Mayhew et al., Methods in Enzymology, Vol. 149, pp. 64-77 (1987), Mayhew et al., Biochimica et Biophysica Acta, Vol. 775, pp. 169-174 (1984), and Cheng et al., Investigative Radiology, Vol. 22, pp. 47-55 (1987).

The liposome composition thus prepared is then subjected to a high pressure impingement emulsifier, such as the Microfluidizer® (Registered Trade Mark), an apparatus available Microfluidics Corporation, a subsidiary of Biotechnology Development Corporation, located in Newton, Massachusetts. The Microfluidizer® emulsifier is described and claimed in U.S. Patent No. 4,855,165. The device consists of a high pressue up to about 16,100 kg/cm$^2$ (23,000 psi) pump and an interaction chamber in which the emulsification takes place. The pump forces the composition into the chamber where the composition is split into two streams which pass at very high velocity through at least two slits and collide resulting in the production of small sized liposomes. As one skilled in the art would recognize, generally multiple passes provide a smaller average liposome size and a narrower particle size distribution. Similarly, higher pressures, with the greater shear and cavitation forces produced thereby, are believed to assist in the formation of smaller average liposome sizes. As one example, a pressure of about 7700 kg/cm$^2$ (11,000 psi) coupled with a total of thirty passes through the Microfluidizer® will produce liposome compositions having a mean liposome size of less than about 50 nm.

Although liposomes having a size of less than 50 nm may be employed in the second embodiment of the subject invention, preferably the liposomes have a minimum size range of about 20 nm, more preferably between 20 and 35 nm, most preferably about 35 nm. It has been found that generally the smaller the liposomes, the greater the relaxivity and/or contrast enhancement and/or the longer the circulation half-life.

The materials which may be utilized in preparing the liposomes of the present invention include any of the materials or combinations thereof known to those skilled in the art as suitable in liposome construction. Such materials include, but are not limited to, lipids such as cholesterol, phosphatidylcholine, phosphatidylethanolamine, phosphatidylserine, phosphatidylglycerol, phosphatidic acid, phosphatidylinositol, lysolipids, fatty acids, sphingomyelin, glycosphingolipids, glucolipids, glycolipids, sulphatides, lipids with ether and ester-linked fatty acids, polymerizable lipids, and combinations thereof. As one skilled in the art would recognize, other nonlipid materials such as polysaccharides and other polymers can be used alone, or in combination with, the lipid materials, in preparing the subject liposomes. These materials can be employed in varying combinations and ratios, in accordance with conventional liposome preparatory protocol. Preferable liposome constructions include constructions containing lipid materials, most preferably those comprising about 60 to about 80 mole percent phosphatidylcholine and about 20 to about 40 mole percent cholesterol. Such constructions are most preferred because of their expected superior stability and relaxivity characteristics.

The liposomes of the present invention encapsulate paramagnetic and superparamagnetic agents. As used herein, the phrase "paramagnetic agent" denotes a compound comprising a transition, lanthanide and actinide element covalently or nonconvalently bound to complexing agents or to proteinaceous macromolecules. The phrase "paramagnetic agent" also denotes stable free radicals. Preferably, the elements are transition and lanthanide elements. Preferable transition and lanthanide elements include Gd(III), Mn(II), Cu-(II), Cr(III), Fe(II), Fe(III), Co(II), Er(II), Ni(II), Eu(III) and Dy(III). Suitable complexing agents will be readily apparent to those skilled in the art and include, but are not limited to, diethylenetriamine- pentaacetic acid (DTPA), ethylenediaminetetraacetic acid (EDTA), 1,4,7,10-tetraazacyclododecane-N,N',N',N'''-tetraacetic acid (DOTA), 1,4,7,10-tetraazacyclododecane-N,N',N''-triacetic acid (DO3A), 3,6,9-triaza-12-oxa-3,6,9-tricarboxymethylene-10-carboxy-13-phenyl-tridecanoic acid (B-19036), hydroxybenzylethylenediamine diacetic acid (HBED), N,N'-bis(pyridoxyl-5-phosphate)ethylene diamine, N,N'-diacetate (DPDP), 1,4,7-triazacyclononane-N,N',N''-triacetic acid (NOTA), 1,4,8,11-tetraazacyclotetradecane-N,N'N'',N'''-tetraacetic acid (TETA). Preferable complexing agents are DTPA and DOTA, most preferably DTPA. Suitable proteinaceous macromolecules include albumin, polyarginine, polylysine, gamma-globulin and beta-globulin. Preferable proteinaceous macromolecules are albumin, polyarginine, polylysine, and polyhistidine. Suitable stable free radicals include stable nitroxides. As used herein, the phrase "superparamagnetic agent" denotes ferro- or ferrimagnetic compounds, such as magnetite. If desired, vasoactive amines may be encapsulated along with the paramagnetic and/or superparamagnetic agents.

Preferably, the liposomes of the present invention encapsulate paramagnetic agents. More preferably, the paramagnetic agents are lanthanide elements bound to complexing agents. Even more preferably, the paramagnetic agents are Gd(III)-DTPA or Gd(III)-DOTA. Most preferably, the paramagnetic agents are Gd-(III)-DTPA.

The present invention is useful in diagnosing the presence of tumerous tissue in a patient. The patient can be any type of mammal, but most preferably is a human. The use of the contrast agent of the invention is particularly useful in diagnosing the presence of tumors of the liver and spleen, although tumors in other organs or other regions such as muscle regions may be diagnosed. Bone marrow is also believed to particularly well suited to the use of this contrast agent.

The liposomes of the present invention are also useful in blood pool imaging, that is, imaging of the blood present in the mammalian system. Thus, the subject liposomes can be employed in assessing the vascularity of both healthy and tumors tissues, and accordingly, are useful in evaluating, for example, the pathological conditions of decreased vascular perfusion such as myocardial or cerebral ischemia. As blood pool imaging agents, the liposomes of the present invention are also useful in the determination of blood volume, and the assessment of vascular leakage (including the assessment of vascular pore size), determinations which may be of importance in the diagnosis and treatment of a variety of diseases including cancer and ischemia.

The diagnostic process using the MRI agents of the present invention may be carried out as follows. First, the patient is administered with liposomes of the invention, that is, lioposomes prepared by a freeze-thaw extrusion process and having encapsulated therein a paramagnetic and/or superparamagnetic agent, and having a diameter of less than about 50 nm.

The patient (either the entire patient or a particular organ or region of that patient), is then scanned using MRI to obtain visible images of any tumorous tissue or the blood pool of a patient.

As one skilled the art would recognize, administration may be carried out in various fashions, such as intravascularly, intralymphatically, parenterally, subcutaneously, intra-muscularly, or intraperitoneally, using a variety of dosage forms. Preferable routes of administration are intravascular and intralymphatical. The useful dosage to be administered and the mode of administration will vary depending upon the age, weight and mammal to be diagnosed, and the particular liposome construction and paramagnetic and/or super-paramagnetic agent employed. Typically, dosage is initiated at lower levels and increased until the desired contrast enhancement is achieved. In carrying out such a diagnostic method with the MRI agenst of the present invention, the liposomes can be used alone in combination with one another, or in combination with other diagnostic and/or therapeutic agents.

The MR imaging techniques employed are conventional and are described, for example, in D.M. Kean and M.A. Smith, Magnetic Resonance Imaging: Principles and Applications (William and Wilkins, Baltimore 1986). Contemplated MRI techniques include, but are not limited to, nuclear magnetic resonance (NMR) and electron spin resonance (ESR). The preferred imaging modality is NMR.

The present invention is further described, by way of illustration only, in the following Examples.

Examples

Example 1

Preparation Of Gd-DTPA-Containing Liposomes By Freeze-Thaw Extrusion Techniques and Characterization of the Same.

Unilamellar vesicles were prepared using the freeze-thaw extrusion process as follows. Egg phosphatidylcholine (0.216 g), cholesterol (0.077 g), and wheat germ digalactosyldiglyceride (0.023 g) (molar ratio 55:40:5) were dissolved in 1 ml chloroform stock solution. Fifty $\mu$l of a $^3$H-DPPC ($^3$H-dipalmitoyl phosphatidylcholine) stock solution (0.25 mCi/2.5 ml toluene, NEN Canada) was added, the mixture evaporated initially under nitrogen, then held at reduced pressure for two hours to remove residual solvent.

The lipid mixture was dispersed in 4 ml 0.67 M $^{153}$Gd-DTPA pH 7.0, to which had been added 50 $\mu$l of $^{14}$C-inulin (0.25 mCi/2.5 ml water, NEN Canada), by vortexing at room temperature to yield multilamellar vesicles (MLVs). The MLVs were transferred to a 4.5 ml cryovial, then subjected to five cycles of freeze-thawing using liquid nitrogen. The frozen and thawed MLVs were then sized by ten passes under nitrogen pressure through two stacked 0.4 micron polycarbonate filters (Nucleopore) using an Extruder Device (Lipex Biomembranes). Two thirds of this sized preparation was then passed ten times through two stacked 0.2 $\mu$ filters. Half of this latter preparation was then passed ten times through two stacked 0.1 $\mu$ filters.

For each of the sized vesicle preparations, external Gd-DTPA was removed by chromatography on Sephadex G50 F (Pharmacia) which had been re-swollen by stirring for two hours at room temperature in saline buffer consisting of 10 mM HEPES, 6 mM KCl, 139 mM NaCl, pH 7.4. G50 F was packed into 23 x 1.5 cm Biorad columns and equilibrated with at least 50 ml of buffer prior to addition of sample. The sample load was 1 ml. Vesicles were eluted with saline buffer in the column void volume and a sample taken for scintillation counting to determine trap volumes as described in Hope et al., Biochimica et Biophysica Acta, Vol. 182, pp. 55-65 (1985). For one batch of 0.4 micron vesicles, non-encapsulated Gd-DTPA was removed by dialysis of the extruded vesicles against 4 liters of saline buffer at 4°C with constant stirring. The external dialysis medium was changed a total of eight times over 48 hours. $^{14}$C-inulin tracer was used to determine encapsulation efficiency.

The size of the 0.4 micron vesicles was assessed by electron microscopy using negative staining techniques.

The lipid and Gd-DTPA concentrations in the different vesicle preparations are shown in Table I. The 0.4 $\mu$ vesicles contained the highest concentration of Gd-DTPA. The 0.4 $\mu$ vesicle preparations had 39% efficiency for encapsulation of Gd-DTPA and removal of free Gd-DTPA by dialysis resulted in the highest concentration of Gd-DTPA. Electron microscopy of the 0.4 $\mu$ vesicles showed a mean diameter of 239 ± 39 nm.

Table I

| Characterization of Freeze-Thaw Gd-DTPA Liposomes | | | |
|---|---|---|---|
| Encapsulated Filter Pore Size (nm) | Lipid Conc. moles/ml | Gd-DTPA Conc. moles/ml | Efficiency (%) |
| 100 | 35 | 38 | 16 |
| 200 | 39 | 42 | 20 |
| 400 | 44 | 47 | 39 |
| 400* | 116 | 124 | 39 |

*Unbound Gd-DTPA removed by chromatography, dialysis used in all other cases.

Example 2

Analysis Of In Vitro Stability Of Gd-DTPA-Containing Liposomes Prepared By Freeze-Thaw Extrusion Techniques.

In vitro stability of the 0.4 $\mu$ liposomes prepared in Example 1 were tested by dialysis performed over 72 hours against normal saline (6 x 1 L) at 4°C. The stability was calculated by counting the $^{153}$Gd-DTPA activity remaining in the liposomes at the end of 72 hours.

The vesicles were found to be 100% stable to the prolonged dialysis.

Example 3

Analysis Of In Vivo Contrast Enhancement And Toxicity Of Gd-DTPA-Containing Liposomes Prepared By Freeze-Thaw Extrusion Techniques.

All in vivo imaging was performed on a Siemens 1.5 Tesla magnet (Iselin, N.J.). Spin echo technique was utilized for both T1- and T2-weighted imaging. T1-weighted sequences utilized parameters TR/TE: 400 msec/16 msec, 4 acquisitions, 256 x 256 matrix 16 cm field view for 2 mm thick slices. For T1 calculations, images were acquired using a fixed TE of 28 msec with TR varying from 300, 500, 1500 and 3500 msec.

10

EP 0 442 962 B1

For T2 calculations a fixed TR of 3500 msec was used with TE values of 28, 45, 70, 90 and 150 msec. T1 and T2 calculations were preformed using the software on the Magnetom System (Iselin, N.J.) by selecting a region of interest on the CRT monitor.

Male buffalo rats, weighing approximately 450 grams, were scanned using a 7.5 cm circular surface coil. The animals were scanned in the supine position, and signal intensities of liver, paraspinal muscle, kidney, renal pelvis, bladder and vascular structures were measured pre and post contrast. Rats were imaged pre and post contrast injection of either 0.4 $\mu$ Gd-DTPA liposomes (8 rats) or free Gd-DTPA (2 rats).

Injections of the 0.4 $\mu$ liposomes prepared in Example 1 were performed by tail vein injection using a 26 gauge butterfly needle. Gd-DTPA liposomes were injected over a period of approximately two minutes; liposomes were diluted with an equal volume of normal saline immediately prior to injection. After imaging, animals were observed for eight weeks for any signs of delayed toxicity.

Scans of rats pre and post contrast with Gd-DTPA liposomes showed enhancement of liver, renal cortex and vascular structures. A dose of 0.1 millimoles/kg or greater of Gd-DTPA in liposomes resulted in a dramatic increase in signal intensity (S.I.) as shown in Table II. Maximal enhancement was observed immediately after injection of contrast agent, but enhancement of liver and vascular structures was sustained for two hours after contrast in two animals imaged two hours after injection of liposomal Gd-DTPA. Scans of two control animals after injection of 0.1 and 0.2 millimoles/kg of free Gd-DTPA showed enhancement of renal cortex, renal pelvis and urinary bladder, but no appreciable enhancement of liver or vascular structures.

During I.V. injection of Gd-DTPA liposomes there was no sign of respiratory distress or other toxicity in the rats. The rats were observed for two months after injection of liposomes and then sacrificed and showed no evidence of toxicity from Gd-DTPA liposomes either at time of injection or on delayed follow-up and gross pathologic examination.

11

Table II

| Dose Gd-DTPA Freeze-Thaw Liposomes and Contrast Enhancement | | | |
|---|---|---|---|
| Dose (mM/Kg) | Liver | Muscle | Liver + Muscle |
| 0.054 lipo (n = 1) | 5.2% | 3.9% | 1.3% |
| 0.080 lipo (n = 1) | 71.1% | 31.0% | 30.6% |
| 0.100 lipo* (n = 5) | 10.0 to 71.1% x = 25.0% | x = -11.6% | x = 39.0% |
| 0.130 lipo (n = 1) | 29.0% | -46.2% | 239.8% |
| 0.254 lipo (n = 1) | 232.0% | 151.4% | 153.2% |
| 0.109 Free Gd-DTPA | 0.0% | 22.4% | -19.3% |
| 0.218 Free Gd-DTPA | 11.6% | 3.1% | 8.2% |
| Liver enhancement: (liver S.I. post - liver S.I. pre) + (liver S.I. pre). Muscle enhancement (paraspinal muscle): (muscle S.I. post - muscle S.I. pre) + muscle pre. Liver + muscle refers to ratio of liver enhancement to muscle enhancement. Post-contrast S.I. measured from scans 30 min after I.V. injection of contrast agents. S.I. = signal intensity, lipo = liposomal Gd-DTPA, x = mean. *Note that 5 rats were injected with dose of 0.1 millimoles Gd-DTPA/kg encapsulated within liposomes and mean values for enhancement are depicted. | | | |

Example 4

Preparation And In Vitro Characterization Of Gd-DTPA-Containing Liposomes Prepared By Freeze-Thaw Extrusion Techniques.

Preparation:

Unilamellar vesicles were prepared by extrusion through polycarbonate filters under moderate pressure using the procedures set forth in Hope et al., Biochimica et Biophysica Acta, Vol. 182, pp. 55-65 (1985). Vesicles were composed of egg phosphatidylcholine (PC), either alone, or in combination with 40 mole percent cholesterol. PC was obtained from Avanti Polar Lipids and cholesterol from Sigma; both were used without further purification. Typically, 3.6 mmoles (2.83 g) of PC and 2.4 mmoles (0.93 g) of cholesterol were dissolved together in a minimum volume of chloroform in a 250 ml round-bottom flask to which was added 50 $\mu$l of $^3$H-dipalmitoyl-phosphatidylcholine (DPPC) stock solution (250 $\mu$Ci/2.5 ml toluene, NEN, Canada). The purpose of the $^3$H-DPPC is to serve as a means of quantitation of the lipid concentration. The chloroform was removed initially by rotary evaporation under reduced pressure to leave a thin film on the walls of the flasks, then the contents held under reduced pressure (< 0.1 mm Hg) for at least two hours to remove residual solvent. The lipid was dispersed by vigorous shaking at room temperature in 20 ml of 0.67 M Gd-DTPA, pH 7 (sodium salt), labeled with 5 $\mu$l of $^{14}$C-inulin (250 $\mu$Ci/2.5 ml water, NEN, Canada). The $^{14}$C-inulin served as a aqueous marker and permitted a facile determination of the trap volumes of the vesicles. The multilamellar vesicles formed upon dispersion were transferred to cryovials then quench-frozen in liquid nitrogen. The cryovials were then placed in warm water until the lipid suspension had completely thawed. This cycle of freezing and thawing was repeated a further four times. The frozen and thawed vesicles were then sized by ten passes under nitrogen pressure (< 100 psi) through two stacked 0.4 micron polycarbonate filters (Nucleopore) using the Extruder Device (Lipex Biomembranes, Canada) to produce liposomes having a mean of about 400 nm. A portion of this sized preparation was then passed ten times through two stacked 0.2 micron filters to produce liposomes having a mean of about 200 nm. A portion of this latter preparation was then passed a further ten times through either two stacked 0.1 or 0.05 micron filters to produce liposomes having means about 100 and 70 nm, respectively. An aliquot of each of the sized preparations was removed for scintillation counting to determine the $^3$H-DPPC and $^{14}$C-inulin content.

For each of the sized vesicles, external untrapped Gd-DTPA was removed by exhaustive dialysis at 4°C against 4 l of saline buffer (10 m moles 4-(2-hydroxyethyl)-1-piperazineethanesulfonic acid (HEPES), 6 mM KCl, 139 mM NaCl, pH 7.4). The external dialysis medium was changed a total of eight times (8 x 4 l) over 2-3 days. The size of the dialyzed particles were determined by quasielastic light scattering using a Nicomp Model 270 particle sizer operating at 634.2 nm by standard cumulants analysis. The entrapment efficiencies of the vesicles were determined by chromatography of an aliquot of the dialyzed vesicles on Sephadex (Registered Trade Mark) G50 F (Pharmacia), equilibrated with saline buffer. Vesicles with entrapped $^{14}$C-inulin was retained on the column. A sample of the eluted vesicles was taken for dual-label scintillation counting as before. Vesicles were stored at 4°C until further use.

Phantoms were prepared by diluting the vesicle preparation with Dulbecco's phosphate buffered saline (PBS) (Gibco), so as to obtain several samples for each vesicle preparation with an effective concentration of Gd-DTPA in the range 0.2-2 mmoles. Approximately 10 ml of each dilution was placed in 20 ml plastic syringes (Becton Dickinson), which were sealed with parafilm to prevent leakage and placed in a custom-built acrylic phantom holder which positioned the syringes with their long-axes horizontal within the bore of the magnet.

Imaging:

Phantoms were imaged using a Siemens 1.5 Tesla Magnetom whole body scanner (Iselin, N.J.) using body coil. For T1 calculations, images were acquired using spin-echo sequences with a fixed TE of 17 msec and TR values of 100, 300, 450, 600, 900, 1200, 1800, 2500 and 3500 msec. Other parameters were 4 acquisitions, 256 x 256 matrix, 30 cm field of view and 10 mm slices.

Vesicle Stability (Retention):

In vitro stability (retention) of the vesicle preparations in saline was determined at various times after preparation by dialysis (Spectrapor 10 mm tubing, M.W. cutoff 12-14,000) over 120 hours against 5 x 11 changes of normal saline at 4°C. The stability was calculated on the basis of either the percentage of $^{153}$Gd-DTPA activity remaining inside appropriately labelled vesicles or by determination of the $^{3}$H-DPPC/$^{14}$C-inulin ratio by scintillation counting. The results are shown in Table III.

EP 0 442 962 B1

## Table III

### Characteristics of Gd-DTPA-Containing Freeze-Thaw PC:Cholesterol (6:4) Liposomes

| Diameter (nm)[1] Mean $\pm$ S.D. | Trap Volume[2] ($\mu$l/mole) | Lipid Conc.[3] ($\mu$mole/ml) | [Gd]eff[4] ($\mu$mole/ml) | Trapping[5] Efficiency (%) | Lipid Dose[6] mg/kg | Percentage[7] Retention | Relaxivity[8] (sec$^{-1}$ x mM$^{-1}$) |
|---|---|---|---|---|---|---|---|
| Free Gd-DTPA | N/A | N/A | N/A | N/A | N/A | N/A | 2.79 |
| 400 $\pm$ 40 | 1.95 | 181 | 236 | 35 | 95 | 100 | 0.42 |
| 200 $\pm$ 30 | 1.17 | 175 | 137 | 20 | 140 | 94 | 0.51 |
| 100 $\pm$ 20 | 0.87 | 185 | 108 | 16 | 214 | 100 | 1.05 |
| 70 $\pm$ 20 | 0.71 | 169 | 79 | 12 | 267 | 100 | 1.60 |

1. Based on quasielastic light scattering.

2. Trap Volume = T = $\mu$l Gd-DTPA per $\mu$mole of lipid.

3. Lipid concentrations (L) are typical values for preparations used in this study.

4. Effective concentration of Gd-DTPA = T x L x [Gd]int) - 1000. In this study [Gd]int was 670 $\mu$moles/ml.

5. Trapping efficiency = [Gd]eff x 100/[Gd]int.

6. Lipid dose = dose of lipid required to give a [Gd]eff of 0.2 millimole/kg. For PC:Cholesterol (6:4) mixtures, the average M.W. was taken as 626.

7. Percentage retention of internal contents after 4 months storage in saline buffer at 4°C.

8. In vitro relaxitivity = sec$^{-1}$ x mM$^{-1}$.

Example 5

Preparation and In Vitro Characterization of Gd-DTPA-Containing Liposomes Having Different Ratios of Egg Phosphatidylcholine and Cholesterol.

Vesicles were prepared substantially as described in Example 4, except that varying amounts of egg phosphatidylcholine and cholesterol were employed.

Imaging was also carried out substantially as described in Example 4, except that in addition to the imaging done as a Siemans 1.5 Tesla Magnetom scanner, imaging was also carried out on a Toshiba MR-50A 0.5 Telsa whole body scanner.

The results are shown in Table IV. The results reveal that the greater the proportion of cholesterol as compared to phosphatidylcholine, the lower the relaxivity and the lower the effectiveness of the liposomes as contrast agents.

## Table IV

### Relaxivity of Gd-DTPA-Containing Freeze-Thaw PC:Cholesterol Liposomes

| Mole% Cholesterol | Average Vesicle Diameter $(mM^{-1})$ | | |
|---|---|---|---|
| | 170 | 100 | 70 |
| 0.5T | Relaxivity $(sec^{-1} \times m\mu^{-1})^*$ | | |
| 0 | 1.399 (.178) | 1.730 (.189) | 2.850 (.426) |
| 10 | 1.294 (.174) | 1.791 (.216) | 2.618 (.334) |
| 20 | 1.405 (.218) | 1.878 ).219 | 2.556 (.286) |
| 30 | 0.885 (.162) | 1.557 (.229) | 2.241 (.261) |
| 40 | 0.807 (.169) | 1.298 (.188) | 1.864 (.214) |
| 50 | 0.549 (.184) | 0.880 (.220) | 1.354 (.215) |
| 1.5% | | | |
| 10 | 1.105 (.060) | 1.697 (.097) | 2.034 (.088) |
| 20 | 1.039 (.074) | 1.627 (.105) | 1.992 (.117) |
| 30 | 0.721 (.054) | 1.209 (.063) | 1.830 (.087) |
| 40 | 0.712 (.065) | 1.017 (.077) | 1.526 (.097) |
| 50 | 0.361 (.059) | 0.794 (.043) | 1.000 (.049) |

*Relaxivity values are expressed as the mean ($\pm$ standard deviation) based on a linear regression to four values of the effective Gd-DPTA concentration.

Example 6

Preparation and Biodistribution Characterization of Gd-DTPA-Containing Liposomes Prepared By Freeze-Thaw Extrusion Techniques.

Preparation:

Unilamellar vesicles were prepared substantially as described in Example 4, except that about 100$\mu$ci of $^{153}$Gd-DTPA was employed as part of the Gd-DTPA used, and no DPPL was used.

Biodistribution:

Male Fischer 344 rats weighing approximately 250 g were used in the biodistribution studies. Rats were anesthetized in all experiments with intramuscular injection of Rompun® (Registered Trade Mark) (Haver, Kansas City), 0.07 ml/250 g body weight plus Ketamine® (Registered Trade Mark) (Parke-Davis, Morris Plains, N.J.), 0.05 ml/250 g body weight. All contrast injections were performed via tail vein over approximately 1 minute.

The biodistribution of both 100 nm and 30 nm diameter lipid vesicles with entrapped Gd-DTPA was examined in the 250 g male Fischer rats. Three to five animals were injected intravenously with a total dose of 0.1 millimole/kg of Gd-DTPA. Rats were sacrificed at 1 hour, 4 hours and 24 hours post-administration. Blood, heart, liver, spleen, muscle, lung, kidney, muscle and fat samples were removed and placed in tared empty gamma counting tubes, reweighed and counted. Results were expressed in terms of percentage total injected dose per gram wet-weight tissue.

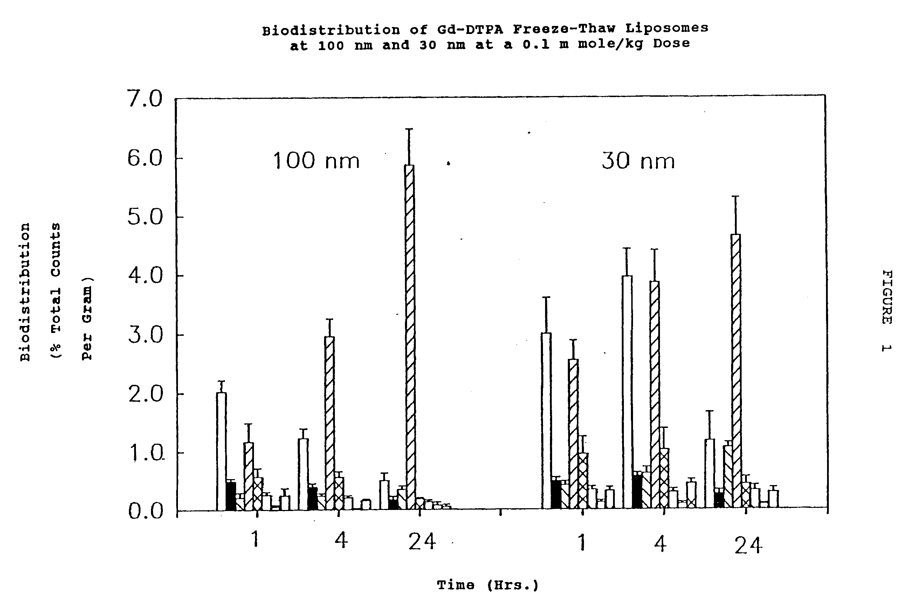

The results are reported in Figure 1. The results shown biodistribution of $^{153}$Gd-DTPA liposomes post-administration for vesicles of 100 nm and 30 nm average diameter given at a dose of 0.1 m mole/kg of Gd-DTPA and $^{153}$Gd-DTPA. The bars in the Figure are grouped in sets of eight at each of the 1, 4 and 24 hour intervals. From left to right at each interval is reported the biodistribution data for the blood, heart, liver, spleen, lung, kidney, muscle, and fat. As the results reveal, the smaller liposomes showed a higher uptake by the liver, spleen, lung, muscle, and bone marrow, and a significantly longer blood pool phase. Thus, the smaller vesicles are superior in terms of biodistribution characteristics.

Example 7

In Vivo Contrast Enhancement Of Hepatic Metastases By Gd-DTPA-Containing Liposomes Prepared By Freeze-Thaw Extrusion Techniques.

Cell Lines and Tumor Model:

The cell line employed, C5, is a clonal derivative of a rat liver epithelial cell line initially derived from the livers of newborn Fisher rats and subsequently transformed in vitro by transfection with a plasmid containing the T24ras gene driven by the mouse metallothionein promotor. See, Li et al., P.N.A.S., Vol. 35, pp. 344 (1988); Reynolds et al., Oncogene, Vol. 323, pp. 323-330 (1987). It has been shown to grow rapidly in vivo in subcutaneous tissue, intraperitoneally and intrahepatically.

The tumors used in this study were generated by injecting 3 x $10^5$ cells into the superficial lobe of rat livers. Three separate injections of $10^5$ cells were made within an approximate area of 0.5 cm$^2$ under direct visualization. The tumors were allowed to grow for 14 to 17 days before imaging followed by sacrifice.

Liposome Preparation:

Egg phosphatidyl choline (EPC) cholesterol (Chol) vesicles (molar ratio 6:4) were prepared by extrusion as described in Example 4.

Imaging:

MR imaging was performed on a whole body 1.5 T Siemens Magnetom (Erlangen, West Germany) using a 15 cm Helmholtz receive only coil. Spin echo technique was used with TR/TE of 400 msec/17 msec, 2 mm slices with 4 acquisitions. Images were obtained pre and post contrast in both axial and coronal planes with post contrast images obtained from 5 to 60 minutes post contrast.

Rats were lightly sedated with ether and then fully sedated with I.P. Ketamine/Rompun 0.10 ml/100 g body weight. Contrast injections were performed by tail vein over two minutes with volume approximately 0.5 ml. Doses were as follows: free Gd-DTPA 0.5 and 0.2 mM/kg, 400 nm Gd-DTPA liposomes 0.5, 0.2 and 0.1 mM/Kg and 70 nm Gd-DTPA liposomes 0.5 and 0.2 mM/Kg with one rat injected with each dose.

Image Analysis:

Pre and post contrast images were separated and, in a blind study, five radiologists experienced in interpreting MR and oncologic imaging were given the MR images to interpret. The MR scans were presented to the radiologists randomly without knowledge of path findings or contrast agent used. The

18

radiologists were instructed to count the number of metastases, plot lesion locations and measure lesion size. The number and sizes of lesions detected were then correlated with the histologic sections and the true number of metastases detected was determined for pre and post contrast scans. The Mann-Whitney test was used to test for statistical significance of results for lesion detection pre and post contrast.

Histology:

Immediately after imaging, the rats were sacrificed by ether overdose and the livers removed and fixed in 10% formalin. The gross liver specimens were photographed and sectioned in the coronal plant at 2 mm intervals. The number of lesions were counted and size of lesions were measured to correlate with findings on MR images. Microscopic sections were prepared using hematoxylin and eosin stain and studied with light microscopy.

Detection of Hepatic Metastases:

The accuracy of detection of lesions pre and post contrast after Gd-DTPA is shown in Table V. Table V shows that liposomal Gd-DTPA caused a statistically significant improvement in lesion detection by the five radiologists. Free Gd-DTPA caused a statistically significant reduction in lesion detection.

**Blind Study Detection of Hepatic Metastases**
**Pre and Post Contrast by 5 Radiologists**

| Dose | #Path Proven | #Pre | #Post | Sensitivity Pre | Sensitivity Post | Accuracy Pre | Accuracy Post |
|---|---|---|---|---|---|---|---|
| 0.5 mm/kg 70 nm | 4 | 1.83+.41 | 4.00+0*** | 45.8+10.2% | 100+0% | 37.5+13.7% | 100+0% |
| 0.2 mm/kg 70 nm | 6 | 4.80+.84 | 6.00+0* | 80.0+13.9% | 100+0% | 80.0+13.9% | 100+0% |
| 0.5 mm/kg 400 nm | 7 | 3.40+.55 | 6.17+.98*** | 48.6+7.8% | 85.7+14.3% | 45.7+11.9% | 85.7+14.3% |
| 0.2 mm/kg 400 nm | 4 | 2.00+1.41 | 2.80+.84 | 50.0+35.3% | 70+20.9% | 50.0+35.3% | 70.0+20.9% |
| 0.1 mm/kg 400 nm | 2 | 1.17+.41 | 2.00+0* | 58.3+20.4% | 100+0% | 58.3+20.4% | 100+0% |
| 0.5 mm/kg free | 7 | 5.00+.63 | 2.80+1.64** | 71.4+10.1% | 40.0+23.5% | 71.4+10.1% | 40.0+23.5% |
| 0.2 mm/kg free | 2 | 1.60+.55 | 0+0*** isointense | 80.0+27.4% | 0+0% | 70.0+27.4% | 0+0% |

Legends: #Path Proven = number of metastases proven on histology, #Pre and #Post refer to number of metastases detected on Pre and Post contrast scans. Sensitivity Pre and Sensitivity Post = percentage of lesions detected on Pre and Post contrast scans respectively. Accuracy Pre and Post include adjust-ment for false positives. Statistical significance: * = p < .05, ** = p < .01, *** = p < .005. Same p values correspond for differences between # of metastases detected Pre and Post contrast as well as sensitivity and accuracy. Note that free Gd-DTPA(free) caused significant reduction in detection of metastases.

Example 8

Preparation Of Gd-DTPA-Containing Liposomes By Microemulsification Techniques And Size Characterization Of The Same.

Unilamellar vesicles were prepared using the microemulsification process as follows. Egg phosphatidylcholine (20 g) and cholesterol (2.46 g) were combined in chloroform. The solvent was removed initially under rotary evaporation to form a thin film on the wall of a round-bottom flask. The flask and contents were then left under reduced pressure (< 0.1 mm Hg) for about 18 hours. The dry lipids were rehydrated in Gd-DTPA (670 mM) by shaking at room temperature to form a dispersion having a concentration of approximately 300 $\mu$moles/ml as measured by lipid phosphorous.

The Microfluidics® M-100 T emulsifier was cleaned by passing approximately 100 ml of ethanol through the device, followed by 200 ml of water, followed by 200 ml of dialysis buffer (10 mM HEPES, 139 mM NaCl, 6 mM KCl, pH 7.4). The pump pressure was set at about 11,000 psi, yielding a flow rate of approximately 200 ml/min, and the lipid dispersion passed through the Microfluidizer® with water cooling. The product was collected and then recycled through the device for a total of 30 passes.

The size distribution of the vesicles was determined by quasielastic light-scattering using a Nicomp Model 270 particle sizer operating at 634.2 nm by standard cumulants analysis.

The results are shown in Table VI. As the results indicate, the majority of the liposomes prepared have a diameter of between about 42.8 and 52.1 nm.

Table VI

| Size Characterization of Microemulsified Gd-DTPA Liposomes | |
| --- | --- |
| d/nm[1] | Relative Area[2] |
| 21.0[3] | 78.6[3] |
| 21.4 | 82.7 |
| 21.8 | 84.7 |
| 42.8 | 29.0 |
| 44.4 | 93.3 |
| 46.1 | 99.9 |
| 48.0 | 76.4 |
| 50.0 | 18.3 |
| 52.1 | 8.8 |
| 171.4[4] | 9.0[4] |
| 200.0[4] | 16.6[4] |
| 240.0[4] | 20.8[4] |
| 300.0[4] | 12.8[4] |
| 400.0[4] | 5.2[4] |
| 600.0[4] | 0.4[4] |

[1]d/nm = liposome diameter in nanometers.
[2]Relative Area = total area, relative to the maximum of 46.1 nm, occupied by liposomes having the specified diameters.
[3]Data is believed to represent a machine artifact and not liposomes.
[4]Data is believed to represent either aggregates of liposomes and/or dust particles.

Example 9

Preparation of Gd-DTPA-Containing Liposomes By Microemulsification Techniques And Analysis Of In Vivo Contrast Enhancement.

Preparation:

Unilamellar vesicles were prepared using the microemulsification process as follows. Egg phosphatidylcholine and cholesterol were combined in a ratio of 8:2 in chloroform. The solvent was removed initially under rotary evaporation to form a thin film on the wall of a round-bottom flask. The flask and contents were then left under reduced pressure (< 0.1 mm Hg) for about 18 hours. The dry lipids were rehydrated in Gd-DTPA (670 mM) by shaking at room temperature to form a dispersion that having a concentration of approximately 300 $\mu$moles/ml as measured by lipid phosphorous.

The Microfluidics® M-100 T emulsifier was cleaned by passing approximately 100 ml of ethanol through the device, followed by 200 ml of water, followed by 200 ml of dialysis buffer (10 mM HEPES, 139 mM NaCl, 6 mM KCl, pH 7.4). The pump pressure was set at about 7,700 Kg/cm$^2$ (11,000 psi), yielding a flow rate of approximately 200 ml/min, and the lipid dispersion passed through the Microfluidizer® with water cooling. The product was collected and then recycled through the device for a total of 30 passes.

The size distribution of the vesicles was determined by quasielastic light-scattering using a Nicomp Model 270 particle sizer operating at 634.2 nm by standard cumulants analysis. Liposomes having a mean diameter of less than about 50 nm were obtained.

Imaging:

All in vivo imaging was performed on a Toshiba MR-50A 0.5 Tesla whole body scanner. Scan parameters were TR = 400 msec TE = 15 msec, 5 mm slice, 1 mm interslice gap, 15 cm field of view with 4 acquisitions using a receive-only surface coil.

Male Wistar rats were scanned in the supine position, and signal intensities of liver, kidney and paraspinal muscle as well as urine, were measured pre and post contrast, with post contrast intervals comprising 15 minutes, 1 hour, 4 hours, 24 hours and 48 hours.

Injections of liposomes prepared as described above were made at concentrations of 0.1, 0.05 and 0.025 mM/kg, based on the weight of the rats, and were carried out by tail vein injection using a 26 gauge butterfly needle. The liposomes were injected over a period of approximately two minutes, and were first diluted with an equal volume of normal saline immediately prior to injection.

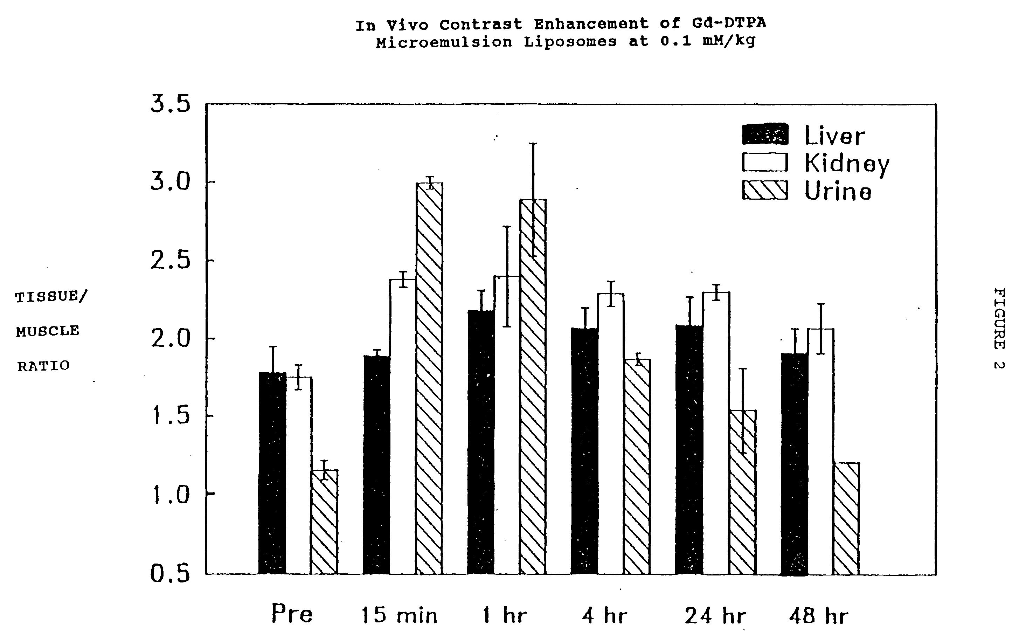

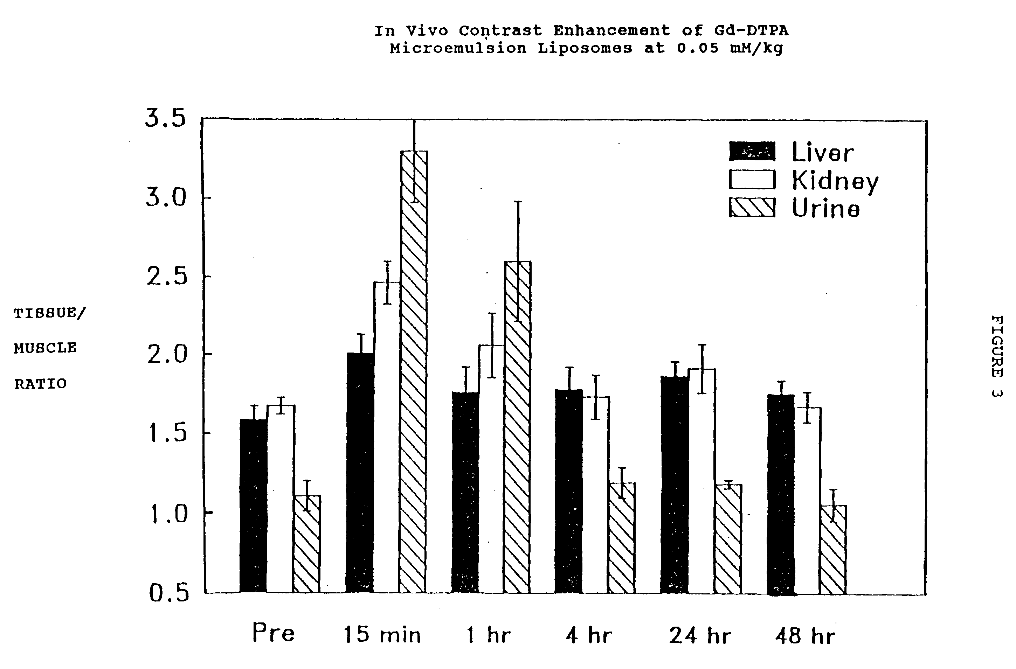

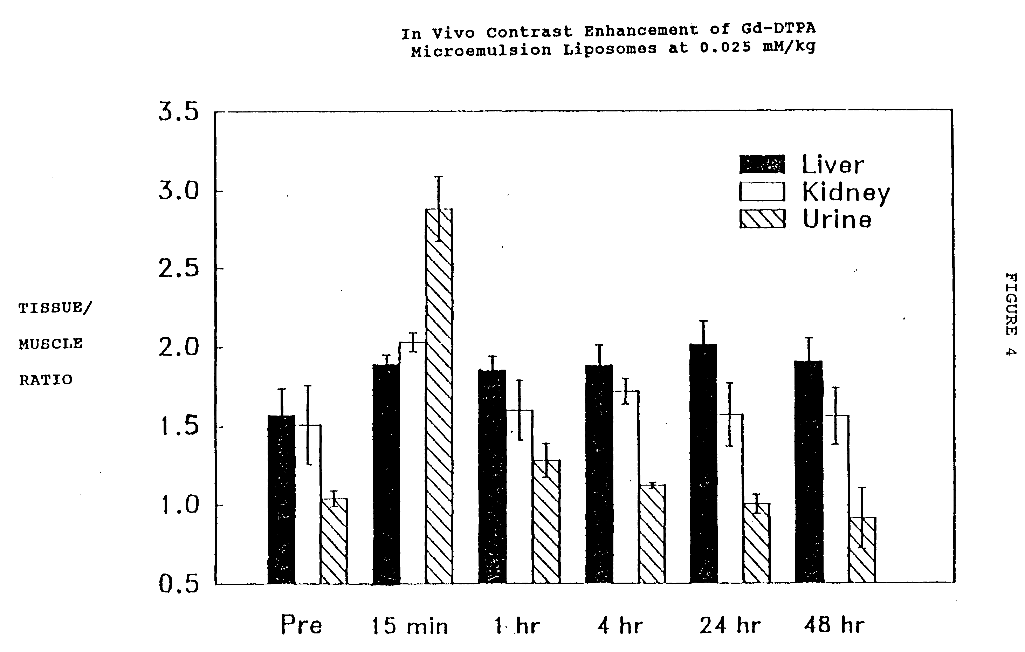

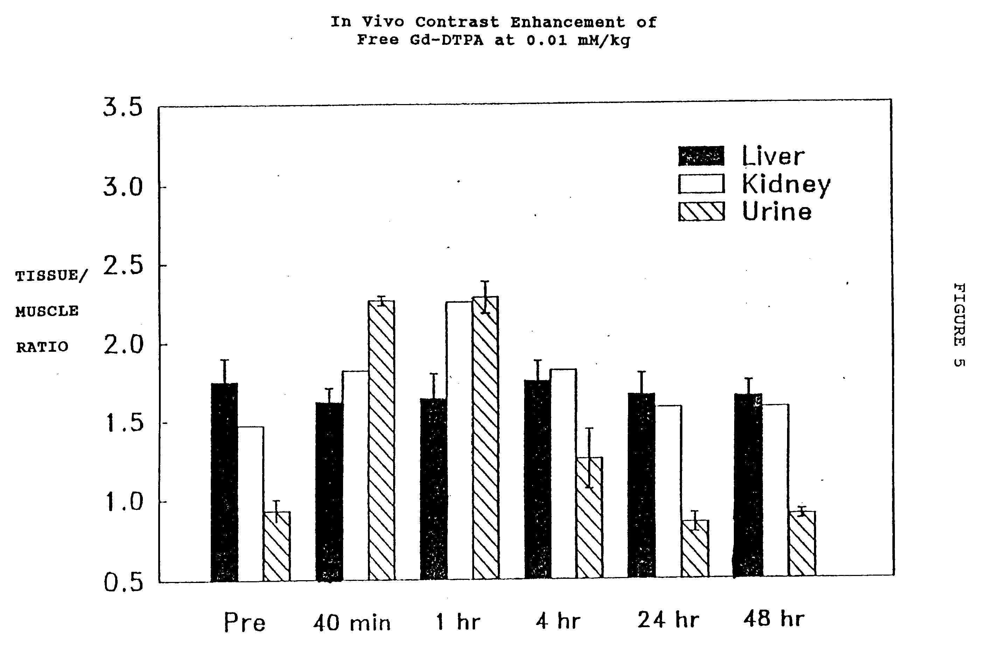

Scans of rats pre and post contrast showed enhancement of the liver, kidney and urine. The results, reported as a tissue to muscle ratio, are shown in Figures 2-4. As a control, scans of rats injected with free Gd-DTPA at a dose of 0.1 mmoles/kg are reported in Figure 5. The results for the control show no significant enhancement of the liver, kidney and urine.

**Claims**

1. The use in the manufacture of an MRI or NMR contrast agent comprising liposomes having encapsulated therein a paramagnetic and/or superparamagnetic agent; characterised in that the liposomes are prepared by a freeze-thaw extrusion process or a microemulsification process and have a diameter of less than 50nm.

2. The use of Claim 1 wherein the contrast agent is an NMR contrast agent.

3. The use in either of Claim 1 or 2 of a liposome prepared by a solvent-free freeze-thaw extrusion process.

4. The use of any preceding Claim wherein said liposomes are lipid materials selected from cholesterol, phosphatidylcholine, phosphatidylethanolamine, phosphatidylserine, phosphatidylglycerol, phosphatidic acid, phosphatidylinositol, lysolipids, fatty acids, sphingomyelin, glycosphingolipids, glucolipids, sulphatides, lipids with ether and ether-linked fatty acids, polymerizable lipids, and combinations thereof.

5. The use of Claim 4 wherein said liposomes are comprised of phosphatidyl choline and cholesterol.

EP 0 442 962 B1

6. The use of Claim 4 or 5 wherein said liposomes comprise 60 to 80 mole percent phosphatidyl choline and 20 to 40 mole percent cholesterol.

7. The use of any preceding Claim wherein said liposomes have a diameter of less than 30nm.

8. The use of any of Claims 1 to 6 wherein said liposomes are selected from the size rage of 20 to less than 35nm.

9. The use of Claim 8 wherein said liposome size range is 20 to 30nm.

10. The use of Claim 8 wherein said liposome size is about 35nm or about 20nm.

11. The use of any of Claims 1 to 10 wherein said paramagnetic agent is a lanthanide or transition element bound to a complexing agent.

12. The use of Claim 11 wherein said lanthanide or transition elements are selected from Gd(III), Mn(II), Cu-(II), Cr(III), Fe(II), Fe(III), Co(II), Er(II), Ni(II), Eu(III) and Dy(III).

13. The use of Claim 11 wherein said complexing agent is selected from diethylene-triamine-pentaacetic acid (DTPA), ethylenediamintetraacetic acid (EDTA), 1,4,7,10-tetraazacyclododecane N,N',N',N'''-tetraacetic acid (DOTA), 1,4,7,10-tetraazacyclododecane-N,N',N''-triacetic acid (DO3A), 3,6,9-triaza-12-oxa-3,6,9-tricarboxymethylene-10-carboxy-13-phenyl-tridecanoic acid (B-19036), hydroxybenzylethylene-diamine diacetic acid (HBED), N.N'-bis (pyridoxyl-5-phosphate)ethylene diamine, N,N'-diacetate (DPDP), 1,4,7-triazacyclononane-N,N',N''-triacetic acid (NOTA) and 1,4,8,11-tetraazacyclotetradecane-N,N'N'', N'''-tetraacetic acid (TETA).

14. The use of Claim 12 wherein said lanthanide element is Gd(III) and said complexing agent is DTPA.

**Patentansprüche**

1. Verwendung von Liposomen für die Herstellung eines MRI oder NMR Kontrastmittels, das Liposomen mit einer darin eingekapselten paramagnetischen und/oder superparamagnetischen Substanz enthält, dadurch gekennzeichnet, daß die Liposomen mittels eines Einfrieren-Auftauen-Extrusions-Verfahrens oder eines Mikro-Emulgier-Verfahrens hergestellt werden und einen Durchmesser von weniger als 50 nm aufweisen.

2. Die Verwendung nach Anspruch 1, wobei das Kontrastmittel ein NMR Kontrastmittel ist.

3. Die Verwendung nach Anspruch 1 oder 2, wobei die Liposomen mittels eines Lösungsmittel freien Einfrieren-Auftauen-Extrusions-Verfahrens hergestellt werden.

4. Die Verwendung nach einem der vorhergehenden Ansprüche, wobei die Liposomen Lipid-Materialien sind, ausgewählt aus Cholesterin, Phospatidylcholin, Phospatidylethanolamin, Phospatidylserin, Phospatidylglycerin, Phosphatidinsäure, Phosphatidylinosit, Lysolipiden, Fettsäuren, Sphingomyelin, Glycosphingolipiden, Glucolipiden, Sulfatiden, Lipiden mit Ether- und Ester-gebundenen Fettsäuren, polymerisierbaren Lipiden und Gemischen von diesen.

5. Die Verwendung nach Anspruch 4, wobei die Liposomen aus Phosphatidylcholin und Cholesterin bestehen.

6. Die Verwendung nach Anspruch 4 oder 5, wobei die Liposomen aus 60 bis 80 Mol% Phosphatidylcholin und 20 bis 40 Mol% Cholesterin bestehen.

7. Die Verwendung nach einem der vorhergehenden Ansprüche, wobei die Liposomen einen Durchmesser von weniger als 30 nm haben.

8. Die Verwendung nach einem der Ansprüche 1 bis 6, wobei die Liposomen ausgewählt sind aus einem Größenbereich von 20 bis weniger als 35 nm.

23

9. Die Verwendung nach Anspruch 8, wobei der Größenbereich der Liposomen 20 bis 30 nm beträgt.

10. Die Verwendung nach Anspruch 8, wobei die Größe der Liposomen etwa 35 nm oder etwa 20 nm beträgt.

11. Die Verwendung nach einem der Ansprüche 1 bis 10, wobei die paramagnetische Substanz ein an einen Komplexbildner gebundenes Element der Lanthaniden oder der Übergangsmetalle ist.

12. Die Verwendung nach Anspruch 11, wobei die Elemente der Lanthaniden oder der Übergangsmetalle ausgewählt sind aus Gd(III), Mn(II), Cu(II), Cr(III), Fe(II), Fe(III), Co(II), Er(II), Ni(II), Eu(III) und Dy(III).

13. Die Verwendung nach Anspruch 11, wobei der Komplexbidner ausgewählt ist aus Diethylen-triamin-pentaessigsäure (DTPA), Ethylen-diamin-tetraessigsäure (EDTA), 1,4,7,10-Tetraazacyclododecan-N,N',N'',N'''-tetraessigsäure (DOTA),1,4,7,10-Tetraazacyclododecan-N,N',N''-triessigsäure (DO3A), 3,6,9-Triaza-12-oxa-3,6,9-tricarboxymethylen-10-carboxy-13-phenyl-tridecansäure (B19036), Hydroxybenzylethylen-diamin-diessigsäure (HBED), N,N'-Bis-(pyridoxyl-5-phosphat)ethylen-diamin, N,N'-Diacetat (DPDP), 1,4,7-Triazacyclononan-N,N',N''-triessigsäure (NOTA) und 1,4,8,11-Tetraazacyclotetradecan-N,N',N'',N'''-tetraessigsäure (TETA).

14. Die Verwendung nach Anspruch 12, wobei das Element der Lanthaniden Gd(III) und der Komplexbildner DTPA sind.

**Revendications**

1. Utilisation dans la fabrication d'un agent de contraste pour IRM ou RMN, comprenant des liposomes dans lesquels est encapsulé un agent paramagnétique et/ou superparamagnétique ; caractérisée par le fait que les liposomes sont préparés par un procédé de gel-dégel - extrusion ou un procédé de microémulsification et ont un diamètre de moins de 50 nm.

2. Utilisation selon la revendication 1, dans laquelle l'agent de contraste est un agent de contraste pour RMN.

3. Utilisation selon l'une des revendications 1 ou 2 d'un liposome préparé par un procédé de gel-dégel - extrusion sans solvant.

4. Utilisation selon l'une des revendications précédentes, dans laquelle lesdits liposomes sont des substances lipidiques choisies parmi le cholestérol, la phosphatidylcholine, la phosphatidyléthanolamine, la phosphatidylsérine, le phosphatidylglycérol, l'acide phosphatidique, le phosphatidylinositol, les lysolipides, les acides gras, la sphingomyéline, les glycosphingolipides, les glucolipides, les sulfatides, les lipides avec des acides gras à liaisons éther et ester, des lipides polymérisables, et des combinaisons de ceux-ci.

5. Utilisation selon la revendication 4, dans laquelle lesdits liposomes sont composés de phosphatidylcholine et de cholestérol.

6. Utilisation selon l'une des revendications 4 ou 5, dans laquelle lesdits liposomes comprennent 60 à 80 pour cent en moles de phosphatidylcholine et 20 à 40 pour cent en moles de cholestérol.

7. Utilisation selon l'une des revendications précédentes, dans laquelle lesdits liposomes ont un diamètre de moins de 30 nm.

8. Utilisation selon l'une des revendications 1 à 6, dans laquelle lesdits liposomes sont choisis dans la plage de dimension allant de 20 à moins de 35 nm.

9. Utilisation selon la revendication 8, dans laquelle ladite plage de dimension de liposome est de 20 à 30 nm.

**10.** Utilisation selon la revendication 8, dans laquelle ladite dimension de liposome est d'environ 35 nm ou d'environ 20 nm.

**11.** Utilisation selon l'une des revendications 1 à 10, dans laquelle ledit agent paramagnétique est un élément des lanthanides ou un élément de transition, lié à un agent complexant.

**12.** Utilisation selon la revendication 11, dans laquelle lesdits éléments des lanthanides ou de transition sont choisis parmi Gd(III), Mn(II), Cu(II), Cr(III), Fe(II), Fe(III), Co(II), Er(II), Ni(II), Eu(III) et Dy(III).

**13.** Utilisation selon la revendication 11, dans laquelle ledit agent complexant est choisi parmi l'acide diéthylène triamine penta acétique (DTPA), l'acide éthylène diamine tétra acétique (EDTA), l'acide 1,4,7,10-tétraazacyclododécane-N,N',N',N'''-tétraacétique (DOTA), l'acide 1,4,7,10-tétraazacyclododécane-N,N',N''-triacétique (DO3A), l'acide 3,6,9-triaza-12-oxa-3,6,9-tricarboxyméthylène-10-carboxy-13-phényl-tridécanoïque (B-19036), l'acide hydroxybenzyléthylène-diamine diacétique (HBED), le N,N'-bis(pyridoxyl-5-phosphate)éthylène diamine, N,N'-diacétate (DPDP), l'acide 1,4,7-triazacyclononane-N,N',N''-triacétique (NOTA) et l'acide 1,4,8,11-tétraazacyclotétradécane-N,N',N'',N'''-tétra acétique (TETA).

**14.** Utilisation selon la revendication 12, dans laquelle ledit élément des lanthanides est Gd(III) et ledit agent complexant est le DTPA.

Biodistribution of Gd–DTPA Freeze–Thaw Liposomes
at 100 nm and 30 nm at a 0.1 m mole/kg Dose

100 nm

30 nm

Biodistribution

(% Total Counts

Per Gram)

Time (Hrs.)

FIGURE 1

EP 0 442 962 B1

26

In Vivo Contrast Enhancement of Gd-DTPA
Microemulsion Liposomes at 0.1 mM/kg

FIGURE 2

EP 0 442 962 B1

In Vivo Contrast Enhancement of Gd-DTPA
Microemulsion Liposomes at 0.05 mM/kg

TISSUE/ MUSCLE RATIO

Liver
Kidney
Urine

Pre    15 min    1 hr    4 hr    24 hr    48 hr

EP 0 442 962 B1

FIGURE 3

FIGURE 4

FIGURE 5

In Vivo Contrast Enhancement of
Free Gd-DTPA at 0.01 mM/kg